# EUROPEAN PATENT SPECIFICATION

(11) **EP 4 132 398 B1**
(45) Date of publication and mention of the grant of the patent: **13.11.2024**
(21) Application number: 20722889.1
(22) Date of filing: 09.04.2020
(51) Int. Cl.: A61B 18/02, A61B 18/08, A61B 18/00, A61B 18/14

(54) **SYSTEMS FOR USING A MULTI-PROBE INTEGRATED ELECTROTHERMAL MODULES (ETMS) DEVICE FOR TUMOR ABLATION**
SYSTEME ZUR VERWENDUNG EINER VORRICHTUNG MIT MEHREREN SONDEN UND INTEGRIERTEN ELEKTROTHERMISCHEN MODULEN (ETMS) ZUR TUMORABLATION
SYSTÈMES D'UN DISPOSITIF ÉLECTROTHERMIQUE INTÉGRÉ MULTI-SONDE (ETMS) POUR ABLATION DE TUMEUR

(43) Date of publication of application: 15.02.2023
(73) Proprietor: Bard Peripheral Vascular, Inc., Franklin Lakes, NJ 07417 (US)
(72) Inventor: SINGH, Aseem, Tempe, Arizona 85281 (US); AKERELE-ALE, Oladipo Peter, Tempe, Arizona 85281 (US); RAMIREZ, Ruben, Tempe, Arizona 85281 (US); MOLL, Eric, Tempe, Arizona 85281 (US); ELDRED, Danielle, Tempe, Arizona 85281 (US)
(74) Representative: Hoffmann Eitle
(86) International application number: PCT/US2020/027369
(87) International publication number: WO 2021/206714

(56) References cited:
- US-A1- 2003 014 095
- US-A1- 2003 014 098
- US-B2- 7 238 184

## Description

### TECHNICAL FIELD

The present specification generally relates to medical devices for treating cancer, and more particularly to medical devices configured and operable for tumor ablation in a treatment area within a patient's body to achieve tumor necrosis.

### BACKGROUND

Common treatments for addressing tumor growth include invasive surgical resection techniques and minimally invasive ablation techniques. Radiofrequency (RF) treatment is an accepted ablation techniques. In general, RF treatment may face difficulties while attempting complete tumor destruction. Further, RF treatment may result in imprecisely defined tumor margins that affect areas of tissue beyond a site targeted for ablation, such as heat transfer to surrounding healthy areas of tissue, along with other potential complications with respect to probe setup within a disease region. Similar complications may occur for other treatment methods that cause direct (thermal) or indirect thermal (microwave) ablation.

Accordingly, a need exists for tumor ablation systems and methods with precise control over a temperature profile at an affected site to enhance tumor necrosis while maintaining excellent tumor margins to ablate unhealthy tissue with respect to the affected site avoiding external areas.
US 7238184 B2 discloses a tissue ablation probe comprising an elongated member, an ablative element mounted on the distal end of the elongated member, and at least one thermoelectric device mounted to the member in thermal communication with the ablative element. The system includes thermal control circuitry for controlling the thermal effect of the thermoelectric device, and an ablation source for supplying ablation energy to the ablative element. A plurality of circumferentially distributed thermoelectric devices are provided, so that radial tissue sectors are selectively affected by independently controlling the thermal effect of the thermoelectric devices. The thermoelectric device(s) are used to cool a heat ablative element. In another example, the thermoelectric device(s) are used to heat an ablative element, thereby forming a heat ablative element. In another example, the thermoelectric device(s) are used to cryogenically cool an ablative element.

### SUMMARY

The invention provides a system for tumor ablation according to claim 1. Embodiments of the invention are defined in the dependent claims.

In one example not falling within the scope of the appended claims, a method for tumor ablation with controlled precision of a temperature profile utilizing a tumor ablation probe device may include disposing a distal end of the tumor ablation probe device in a tissue, the distal end comprising a plurality of electrothermal modules (ETMs) proximally disposed on a device surface. Each ETM may include a first surface component and a second surface component opposite and electrically connected to the first surface component. The method may further include supplying, via a circuit controller communicatively coupled to the tumor ablation probe device, one of a first voltage of a first polarity and a second voltage of a second polarity opposite the first polarity to at least one ETM of the plurality of ETMs. When the first polarity is supplied, the at least one ETM heats the first surface component and cools the second surface component, and when the second polarity is supplied, the at least one ETM cools the first surface component and heats the second surface component. The method may further include repeatedly alternating, via the circuit controller, between the first polarity and the second polarity using on a time sequence cycle. Each ETM may be configured for independent control by the circuit controller.

In another example not falling within the scope of the appended claims, a method for tumor ablation with controlled precision of a temperature profile utilizing a tumor ablation probe device may include disposing a distal end of the tumor ablation probe device in a tissue, the distal end comprising at least one electrothermal module (ETM) on a first probe arm and at least one ETM on a second probe arm, and supplying, via a circuit controller communicatively coupled to the tumor ablation probe device, one of a first voltage of a first polarity and a second voltage of a second polarity opposite the first polarity to the at least one ETM on the first probe arm, the at least one ETM on the second probe arm, or both, as one or more voltage-supplied ETMs. Each ETM may include a first surface component and a second surface component opposite and electrically connected to the first surface component. When the first polarity is suppled, the one or more voltage-supplied ETMs respectively heats the first surface component and cools the second surface component. When the second polarity is supplied, the one or more voltage-supplied ETMs respectively cools the first surface component and heats the second surface component. The method may further include repeatedly alternating, via the circuit controller, between the first polarity and the second polarity using on a time sequence cycle. Each of the first probe arm and the second probe arm may be configured for independent control by the circuit controller.

These and additional features provided by the embodiments described herein will be more fully understood in view of the following detailed description, in conjunction with the drawings.

### BRIEF DESCRIPTION OF THE DRAWINGS

The embodiments set forth in the drawings are illustrative and exemplary in nature and not intended to limit the subject matter defined by the claims. The following detailed description of the illustrative embodiments can be understood when read in conjunction with the following drawings, where like structure is indicated with like reference numerals and in which:
FIG. **1** is a side perspective view of a tumor ablation probe device with multiple probes including integrated electrothermal modules (ETMs), according to one or more embodiments shown and described herein;
FIG. **2** is a side view of the tumor ablation probe device of FIG. 1 being used to treat a tumor, according to one or more embodiments shown and described herein;
FIG. **3** is a side elevation view of an embodiment of the tumor ablation probe device of FIG. **1****,** according to one or more embodiments shown and described herein;
FIG. **4** is a side view of another embodiment of a catheter of another tumor ablation probe device including balloon openings, according to one or more embodiments shown and described herein;
FIG. **5** is a side view of a needle with balloons including ETMs to use with the catheter of FIG. **4****,** according to one or more embodiments shown and described herein;
FIG. **6** is a side view of another tumor ablation probe device including a needle with integrated ETMs mounted thereon, according to one or more embodiments shown and described herein;
FIG. **7** is a side view of another tumor ablation probe device including a ribbed cone with integrated ETMs, according to one or more embodiments shown and described herein;
FIG. **8** is a side view of a catheter including an opening from which the ribbed cone of FIG. **7** is configure to flare out or retract into, according to one or more embodiments shown and described herein;
FIG. **9** is a perspective view of an integrated electrothermal module (ETM), according to one or more embodiments shown and described herein;
FIG. **10** is a flowchart of a process for using the tumor ablation probe device systems of FIGS. **1-9****,** according to one or more examples not falling within the scope of the appended claims;
FIG. **11** is a flowchart of another process for using the tumor ablation probe device systems of FIGS. **1-9****,** according to one or more examples not falling within the scope of the appended claims; and
FIG. **12** schematically illustrates a system for implementing computer and software based methods to utilize the tumor ablation probe device systems of FIGS. **1-9****,** according to one or more embodiments shown and described herein.

### DETAILED DESCRIPTION

Referring generally to the figures, embodiments of the present disclosure are directed to systems for tumor ablation utilizing tumor ablation probe devices as described herein. Various embodiments of such systems are described in detail herein.

Tumor ablation is a minimally invasive procedure that may be used to treat tumors such as of the liver, lung, kidney, and bone. During tumor ablation, thermal energy is used to heat or cool surrounding tissue to cytotoxic levels such as less than -40 degrees Celsius or greater than 60 degrees Celsius. Tumor ablation may use modalities such as radiofrequency (RF), thermal, microwave, chemical treatment, interventional radiology, laser, high-intensity focused ultrasound, localized radiation, cryoablation using extreme cold to destroy tissue (e.g., cause tissue necrosis) associated with a tumor, and irreversible electroporation. Such treatments may not achieve complete tumor destruction, particularly when tumor margins are not well defined. Furthermore, complications may occur from ablation of surrounding healthy tissue.

For the tumor ablation probe devices described herein, electrothermal modules (ETMs) are integrated at a distal end of a probe or catheter and, via thermal modulation (e.g., switching between heating/cooling by the same device), tumor necrosis is achieved within modulated tumor ablation margins. ETMs may be integrated on multiple probes housed within an overall needle/probe design. Such individual, individually movable, and individually controlled probes are advanced into the tumor and, when activated, invoke a heat and cryo cycling to cause tumor necrosis within precise tumor margins.

The ETMs may be thermoelectric generators, such as Seebeck generators. Seebeck generators convert temperature differences directly into electrical energy (e.g., through a Seebeck effect phenomenon in which a temperature differential between two electrically connected junctions produces an electromagnetic force between the junctions). Seebeck generators may operate in reverse such that applying a voltage to the device can cause it to act as a heater or cooler, depending on the magnitude and polarity of the voltage (e.g., though a Peltier effect phenomenon in which voltage applied across two electrically connected junctions produces a temperature differential between the junctions).

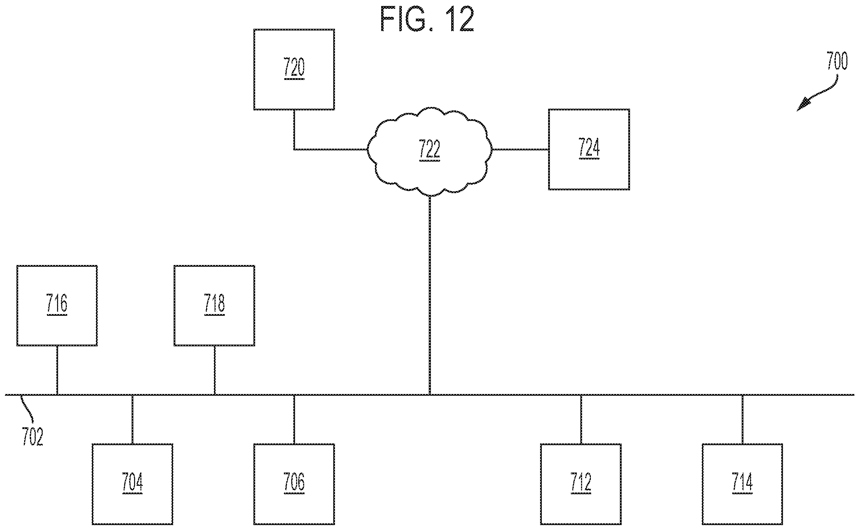

ETM integrated devices included in the devices, systems, and methods as described herein may precisely control an amount of heat generated and provide a rapid thermal/cooling cycling directly to a tumor to enhance tumor necrosis while maintaining tumor margins through precision temperature control on a focused area and while minimizing a risk to surrounding healthy tissue. The tumor ablation probe devices described herein may include a plurality of independently movable and controllable probe arms, each including one or more ETMs made of p-n couples and associated circuitry (e.g., through a processor **704** as described in greater detail below with respect to FIG. **12****)** configured to repeatedly alternate between hot and cold to cause tumor necrosis within precise, temperature controlled margins. For example, the ETMs may be placed around probe arms of a probe device, which probe arms may individually extend into a tumor region. In embodiments, the ETMS may be spaced about probe arms of the probe device, integrated onto probe arms of the probe device, or combinations thereof. A handle may track the depth to which a probe is inserted into the tumor.

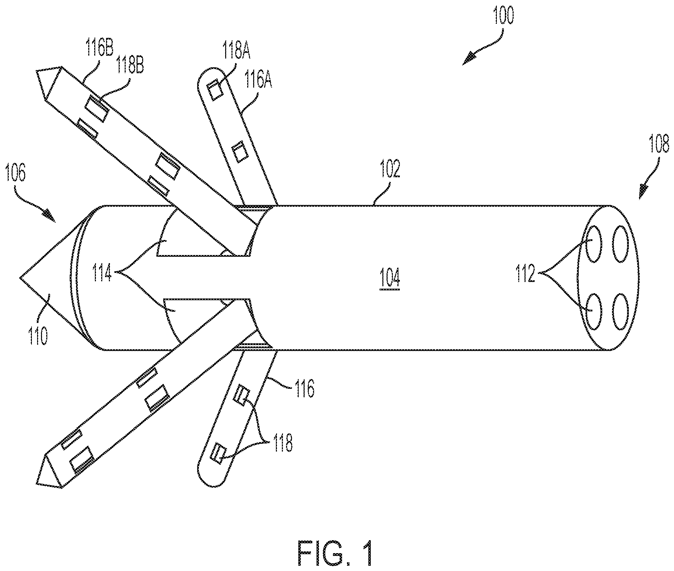

Referring to FIG. **1****,** a tumor ablation probe device **100** includes a probe **102** having an intermediate surface **104,** a distal end **106,** and a proximal end **108.** The distal end **106** includes a tip **110** that is configured to pierce into a tumor. The intermediate surface **104** includes a plurality of openings **114** defined thereon. While the plurality of openings **114** are shown as concentrically and evenly spaced about the intermediate surface **104,** the plurality of openings **114** may be disposed at uneven and/or staggered locations about the intermediate surface **104.** Alternatively, the tumor ablation probe device **100** may include one opening **114.** The proximal end **108** includes a plurality of apertures **112** configured to receive and house a respective plurality of probe arms **116.** Each probe arm **116** is respectively configured to extend from or retract into each opening **114** of the intermediate surface **104** individually and/or independently.

Each probe arm **116** includes one or more integrated electrothermal modules (ETMs) **118.** Each probe arm **116** may include a distal pierce portion configured to pierce a portion of a tumor. In embodiments, the tumor ablation probe device **100** may be semi-rigid, rigid, catheter based, or a similar type that is controlled via a handheld device including a controller circuit (e.g., through a processor **704** as described in greater detail below with respect to FIG. **12****)** configured to switch polarity of input power to provide to the integrated ETMs **118.** Such a repeatedly altered switching of polarity on a predetermined time sequence cycle corresponds to and affects a pre-determined cycling of heating and cooling cycles of the ETMs **118** that are introduced into a tumor site in a minimally invasive manner to result in tissue necrosis. The tumor ablation probe device **100** may be battery powered and/or may utilize a voltage supplier to provide the input power.

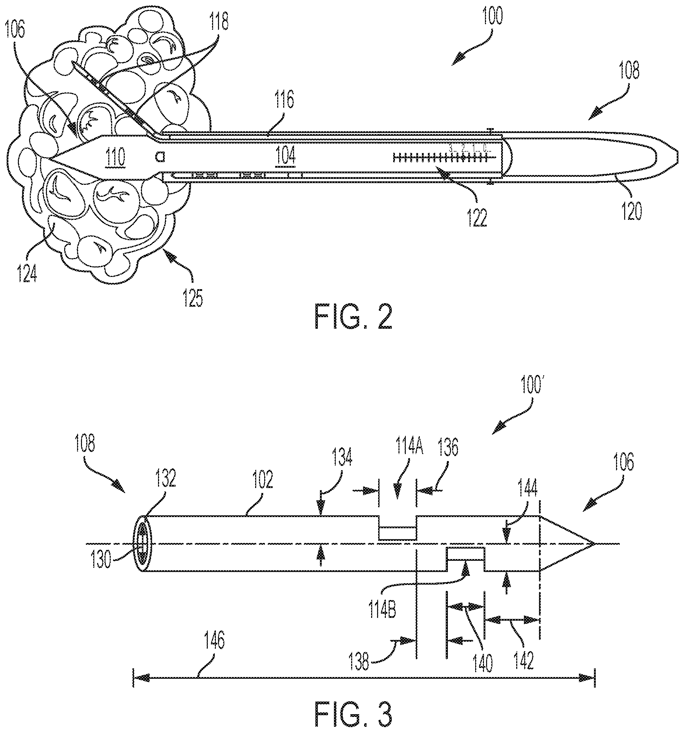

Referring to FIG. **2****,** the tumor ablation probe device **100** is shown as being advanced into a tumor **124** at a tumor site **125.** The tumor ablation probe device **100** further includes a handle **120** including a metric display component **122** to track the advancement or retraction of a probe arm **116** including ETMs **118** into the tumor **124** when the tip **110** is disposed within the tumor **124.** The metric display component **122** may measure such an advancement in centimeters, millimeters, inches, or other customary unit of length. Thus, the metric display component **122** provides a measurement system that may be associated with each individual probe arm **116** to determine a length the probe arm **116** has been extended or retracted. In embodiments, one or more metric display components 122 may be included on the tumor ablation probe device 100. By way of example, and not as a limitation, each probe arm 116 may include a respective metric display component 122.

Referring to FIG. **3****,** an embodiment of a tumor ablation probe device **100',** similar to the tumor ablation probe device **100** except for differences described herein, is shown. The tumor ablation probe device may include a plurality of openings **114** that include staggered openings **114A** and **114B.** In an embodiment, the tumor ablation probe device **100'** includes at the proximal end **108** an opening with a diameter **130** of 6.0 mm and a wall thickness **132** of 1.0 mm. The opening with the diameter 130 of the probe device 100' extends into the probe device 100' as an inner lumen, which also includes a diameter of 6.0 mm. The tumor ablation probe device **100'** further includes a first half portion **134** and a second half portion **144** of the intermediate surface **104** separated along a longitudinal axis. The staggered openings **114A** and **114B** include respectively opening lengths **136, 140** that may be of 4.5 mm each and staggered by a distance **138** of 2.0 mm between nearest ends. Respective probe arms 116 (FIG. 2) may be configured for extension through respective staggered openings 114A, 114B. The staggered openings 114A, 114B may be longitudinally staggered, angularly staggered, axially staggered, radially staggered, or combinations thereof. The staggered openings 114A, 114B may be angularly offset from one another, such as at a 180 degree offset. The staggered openings 114A, 114B may be configured with a size that is less than half of the circumference of the probe device 100'. In an embodiment, the staggered opening **114B** closest to the distal end **106** is spaced at a distance **142** of 10 mm from the beginning of the tip **110** of the distal end **106.** The tumor ablation probe device **100'** may include a length **146** between the proximal end **108** and the tip **110** of the distal end **106** of 200 mm.

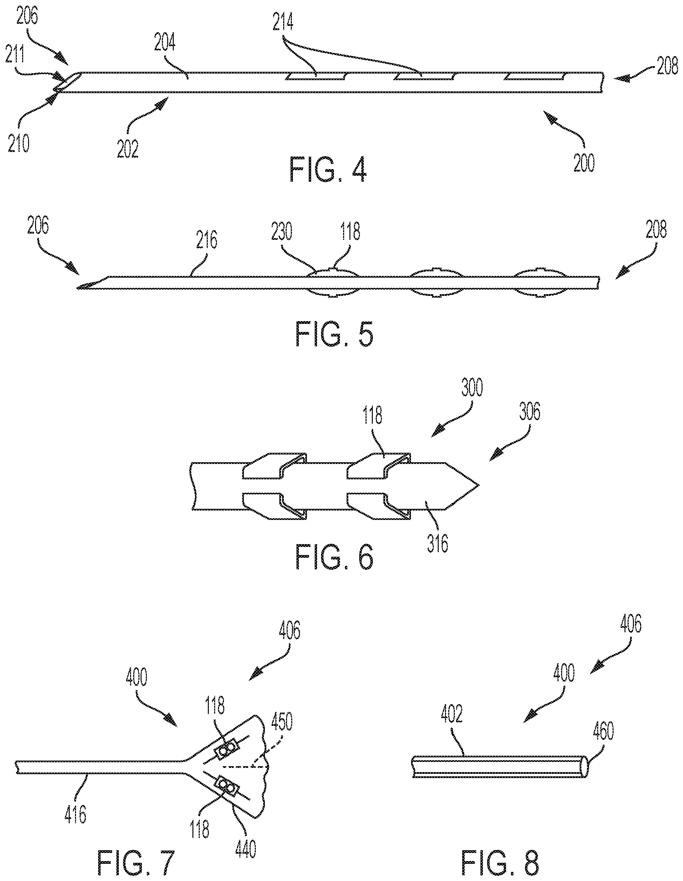

Referring to FIGS. **4** and 5, ETMs **118** are attached to balloons **230** that are configured to expand out through respective balloon openings **214** of a tumor ablation probe device **200.** The tumor ablation probe device **200** illustrated in FIG. **4** includes a probe **202** to encase a needle **216** (e.g., as an embodiment of a probe arm) of FIG. **5****.** It is to be understood that reference to a needle within this disclosure is a reference to a probe arm as described herein, as the needle is an embodiment of the probe arm. The needle **216** includes a plurality of balloons **230** on which one or more ETMs **118** are disposed. While six balloons **230** are shown in the embodiment of FIG. **5** evenly disposed on opposite sides, it is understood within the scope of this disclosure that a different number of balloons and/or balloons having a different spacing may be utilized. In embodiments, approximately two to five ETMs **118** may be placed at various locations of each balloon **230.** The needle **216** may be made of stainless steel. The probe **202** may be alternatively be a catheter. The probe **202** may be made of stainless steel.

The probe **202** includes an intermediate surface **204** disposed between a distal end **206** of the probe **202** and a proximal end **208** of the probe **202.** The intermediate surface **204** includes a plurality of balloon openings **214** sized and shaped to permit advancement and/or retraction of each respective balloon **230** of the needle **216.** The distal end **206** includes a tip **210** extending from the intermediate surface 204. A needle housing opening **211** is defined by an edge of the intermediate surface **204** and at least a portion of the tip 210 and extends as an inner lumen between the distal end **206** and the proximal end **208.** The needle housing opening is configured to house the needle **216** in a position such that **the** plurality of balloons **230** are aligned with the respective plurality of balloon openings **214.** In embodiments, a tip of the needle 216 may extend beyond an end of the tip 210, may be movable within the needle housing opening 211, may still within the needle housing opening 211, or combinations thereof.

During use, the tumor ablation probe device **200** is advanced into a tumor at a tumor site. When the balloons **230** are inflated at a pressure, the balloons **230** advance through respective balloon openings **214,** and the ETMs **118** on each balloon **230** thus protrude along with the balloons **230** through the plurality of balloon openings **214** to sit flush with the tumor at the tumor site. Electrical energy is supplied to the ETMs **118** as described in greater detail below such that the ETMs **118** process through a heating/cooling cycle that is manually, automatically, or partially automatically controlled. Such localized heating/cooling causes necrosis of the tumor or tissue that the ETMs **118** directly contact in a precisely controlled manner within desired tumor margins. Thus, precise control over a temperature profile at an affected tumor site may be achieved through the tumor ablation probe devices described herein, such as the tumor ablation probe device **200.** The tumor ablation probe devices described herein are suitable for minimally invasive use to further more effectively provide precisely controlled tumor necrosis. Upon release of pressure, the plurality of balloons **230** deflate to retract back into the plurality of balloon openings **214** along with the ETMs **118.**

Referring to FIG. **6****,** in another embodiment, ETMs **118** are mounted to a needle **316** near and proximal to a distal end **306** of a tumor ablation probe device **300.** When the tumor ablation probe device **300** is advanced into a tumor such that the ETMs sit flush with the tumor, energy supplied to the ETMs **118** provides a heating/cooling cycle as described further below to cause necrosis of the tumor.

Referring to FIGS. **7** and **8****,** ETMs **118** are mounted on a ribbed cone **440** that is configured to flare out from or retract into an opening **460** of a catheter **402** (e.g., as an embodiment of a probe) of a tumor ablation probe device **400 at** a distal end **406.** In embodiments, the ETMs 118 may be mounted on an external surface of the ribbed cone 440, an internal surface of the ribbed cone 440, to extend through opposing surfaces of the ribbed cone 440, or combinations thereof. As shown in FIG. 7, the ribbed cone **440** is disposed at a distal end of a needle **416,** which is configured to be slidable within the catheter **402** of FIG. **8****.**

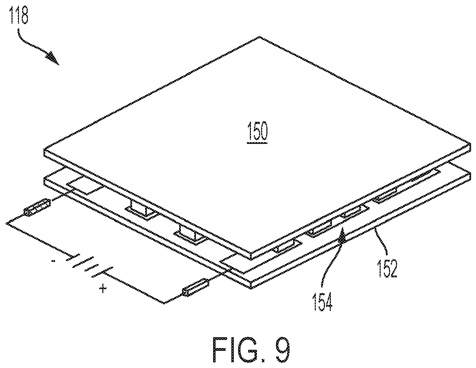

Referring to FIG. **9****,** an exemplary ETM **118** is illustrated. The ETM **118** includes a first surface component **150** and a second surface component **152** opposite the first surface component **150.** The first surface component **150** and the second surface component **152** may be made of ceramic or other suitable conductive material. Electrical junctions as p-n coupled are disposed between the first surface component **150** and the second surface component **152** to energize the surface components **150, 152** through circuitry that sends current to the surface components **150, 152.** In embodiments, the ETMs **118** are semiconductor modules including p-n couples **154** as the electrical junctions. The p-n couples are configured so that a supplied first polarity results in heating of one side/surface area such as the first surface component **150** and cooling of the other side such as the second surface component **152,** and a supplied second polarity opposite the first polarity results in cooling of the first surface component **150,** for example, and heating of the second surface component **152,** for example. Such p-n couples **154** connect the electrically connected surface components **150, 152** through an interface including a p-type semiconductor material and an n-type semiconductor material that allows electrical current to pass through in a direction controlled, in the present disclosure, by an associated polarity. In embodiments, circuitry may be employed to control cycling and temperature ranges of the heating and the cooling. By way of example, and not as a limitation, the first surface component **150** may be heated to a temperature from about 45 degrees Celsius to about 50 degrees Celsius while the second surface component **152** is cooled. In an embodiment, the second surface component may be cooled to about -10 degrees Celsius. Then, the polarity may be reversed to cause the first surface component to cool to about -10 degrees Celsius and the second surface component **152** to heat. In an embodiment, the second surface component **152** may be heated to a temperature from about 45 degrees Celsius to about 50 degrees Celsius. The polarity may then be reversed again to heat the first surface component **150** to a temperature from about 45 degrees Celsius to about 50 degrees Celsius while the second surface component **152** is cooled. The polarity may be reversed any number of times sufficient to provide the desired clinical outcome. The polarity reversals may occur with a frequency of a time sequence cycle as determined by the operator to provide the desired clinical outcome. In example embodiments, the polarity reversals may occur at a frequency of the time sequence cycle defined by a switching period of approximately 2 seconds between each polarity reversal.

The tumor ablation probe devices described herein include a controller circuit configured to switch, such as via a relay or switch board, input power polarity to be directed to the ETMs **118** to control the temperature profile through controlled repeatedly alternation between heating and cooling cycles. The controller circuit is configured to control a pre-determined cycling of heating and cooling on the ETMs **118** that are introduced to a tumor site to result in a desired clinical outcome such as tissue/tumor necrosis of the area the ETMs **118** contact through the heating/cooling cycle switching, for example. The controller circuit may be pre-programmed or manually controlled to switch the input voltage polarity that is delivered to the ETMs **118** to result in a corresponding pre-determined cycling of heating and cooling on the opposing surface components **150, 152** of the ETMs **118.** The ETMs **118** may be individually controlled and set to output different temperatures at each ETM **118** based on a received input voltage polarity supplied by the controller circuit. Thus, tumor ablation within predetermine threshold ranges of a desired area of a tumor may be achieved over temperature profiles of the ETMs **118** to obtain tumor ablation at tumor margins within the predetermine threshold ranges with respect to specific tumor locations, resulting in controlled localized tumor ablation at controlled tumor margins.

In an embodiment, a switch is configured cause a change in a polarity of a provided voltage. In an embodiment, a positive +5V input, for example, may be switched to a negative -5V input, for example, after a controlled time sequence cycle. It is contemplated and within the scope of this disclosure that other voltage magnitudes are possible. It is further contemplated and within the scope of this disclosure that a switch in polarity may switch between different voltage magnitudes rather than opposing polarities of the same voltage magnitude. A resulting temperature of the first surface component **150** or the second surface component **152** of the ETM **118** is at least partially based on the voltage magnitude. The controlled time sequence cycle may be in a range of about every 2 seconds to about every 5 seconds. In such a scenario, the positive +5V causes the ETM **118** to heat the first surface component **150** to provide heating to the tumor and causes the ETM **118** to heat the second surface component **152** to provide cooling to the tumor, while the negative -5V causes the ETM **118** to cool the first surface component **150** to provide cooling to the tumor and causes the ETM **118** to heat the second surface component **152** to provide heating to the tumor.

The tumor ablation probe devices **100, 200, 300, 400** as described herein may include a probe arm such as a needle, catheter, or a hypotube. The various components of the tumor ablation probe devices, such as the probes **102, 202, 402** and probe arms **116, 216, 316, 416** described herein may be made of a metal, a metal alloy, a polymer, a 3D printed material, or combinations thereof. A base probe of the tumor ablation probe device may include multiple tiers of ETM **118** branches along a length and/or around a circumference, such as shown for the probe arms **116** of FIG. **1****.** The tumor ablation probe device may be disposable or non-disposable. The tumor ablation probe device may be battery powered such that power is provided via an internal battery and/or powered via an external electrical component to supply voltage that is electronically coupled to the tumor ablation probe device.

The tumor ablation probe devices as described herein are configured to precisely control an amount of heat generated as well as to provide a rapid heating/cooling cycling. Such configurations enhance tumor necrosis while maintaining excellent tumor margins. For instance, the area of the tissue of the tumor to which such rapid heating/cooling is being applied as described herein via a respective ETM **118** is affected based on a surface area of the first surface component **150** or the second surface component **152** of the ETM **118** providing said heating/cooling while being disposed within and flush against said affected tissue. An amount or magnitude as well as polarity of the voltage supplied to the first surface component **150** or the second surface component **150** of a respective ETM **118** as well as a configuration of one or more p-n couples disposed between the first surface component **150** and the second surface component **150** of the respective ETM **118** as described herein are parameters contributing to the rapid heating/cooling cycling to cause tumor necrosis within desired tumor margins. Thus, the devices described herein provide precision temperature control through control of at least a magnitude and direction of an applied voltage to the ETMs **118** of the devices and increased accuracy of a lethal tumor necrosis zone area as a result of such precision temperature control. The resulting area of the lethal tumor necrosis zone is at least in part determined by the temperature profile established by the precision temperature control, which is controlled by a magnitude and polarity of an applied voltage to the ETMs **118** during a time sequence cycle and includes a repeated alternation between voltage polarities. The controlled temperature profile is applied to a tumor via the one or more ETMs **118** as described herein to accurately cause the lethal tumor necrosis in a zone area within precise tumor margins. Such increased accuracy more effectively ablates a tumor within in a desired zone area while not ablating undesired areas that may be disposed near the desired zone area. The increased precision permits for reproducibility of such results within tight error margins.

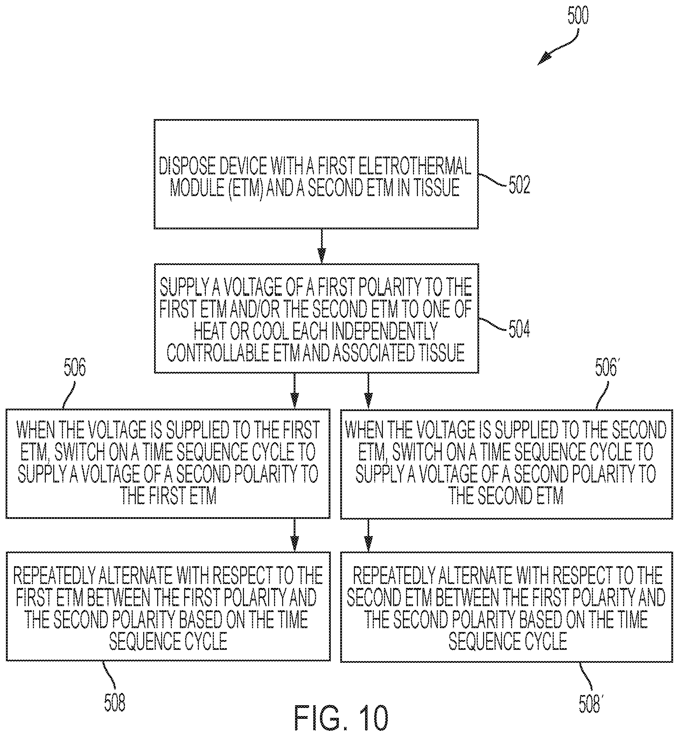

FIG. **10** illustrates a process **500** to use with the tumor ablation probe devices **100, 100', 200, 300, 400** described herein with reference to FIGS. **1-9****.** The process **500** includes a method for tumor ablation with controlled precision of a temperature profile utilizing a tumor ablation probe device **100, 100', 200, 300, 400.** In block **502,** a distal end **106, 206, 306, 406** of the tumor ablation probe device **100, 100', 200, 300, 400** is disposed in a tissue **124.** By way of example and not as a limitation, the distal end **106, 206, 306, 406** includes, or proximally includes, at least two electrothermal modules (ETMs) **118** of a plurality of ETMs on a device surface, each ETM **118A** and **118B** (FIG. **1****)** including a first surface component **150** and a second surface component **152** (FIG. **9****)** opposite and electrically connected to the first surface component **150.**

In block **504,** one of a first voltage of a first polarity and a second voltage of a second polarity opposite the first polarity is supplied via a circuit controller communicatively coupled to the tumor ablation probe device **100, 100', 200, 300, 400** to at least one ETM **118A** or **118**B of the plurality of ETMs **118.** In embodiments, when the first polarity is supplied, the at least one ETM heats the first surface component **150** and cools the second surface component **152,** and when the second polarity is supplied, the at least one ETM cools the first surface component **150** and heats the second surface component **152.** Each ETM **118** may be configured for independent control by the circuit controller.

In blocks **506, 506',** the other of the first voltage of the first polarity and the second voltage of the second polarity is supplied via the circuit controller to at least one ETM **118A** or **118B** of the plurality of ETMs **118** to switch the polarity for a repeated alternation between polarities based on a time sequence cycle. In blocks **508, 508'** the voltage is switched with respect to the respective first or second ETM 118A, 118B with the other of the first voltage of the first polarity and the second voltage of the second polarity based on the time sequence cycle. In an embodiment, the time sequence cycle may include a first time sequence cycle associated with a heating stage of the ETM **118** and a second time sequence cycle associated with the cooling stage of the ETM **118** and different from the first time sequence cycle. In either embodiment, whether the first time sequence cycle and the second tiem sequence cycle are the same or different, the process **500** involves switching between the first polarity and the second polarity via the circuit controller based on the time sequence cycle. As a non-limiting example, the time sequence cycle is in a range of about 2 seconds to about 5 seconds, and when the first polarity is supplied, the at least one ETM **118A** heats heat the first surface component **150** to a range of about 45 degrees Celsius and about 50 degrees Celsius and cools the second surface component **152.** The second surface component **152** may be cooled to about -10 degrees Celsius. When the second polarity is supplied, the at least one ETM **11A** cools the first surface component **150** to about -10 degrees Celsius and heats the second surface component **152.** The second surface component **152** may be heated to the range between about 45 degrees Celsius and about 50 degrees Celsius.

The distal end **106, 206, 306, 406** may include, or proximally include, at least one ETM **118A** on a first probe arm **116A** and at least one ETM **118B** on a second probe arm **116B** (FIG. **1****),** and each of the first probe arm **116A** and the second probe arm **116B** may be configured for independent control by the circuit controller. In embodiments, the ETMs 118 on a same probe arm 116 may be independently controlled and/or ETMs 118 on different probe arms 116, such as the first and second probe arms 116A, 116B, may be independently controlled. The first polarity may be positive such that the second polarity is negative. Alternatively, the first polarity may be negative such that the second polarity is positive. The first voltage may be equal to the second voltage, or the first voltage may be different from the second voltage.

In embodiments, one of the first voltage of the first polarity and the second voltage of the second polarity is supplied to one ETM **118A, 118B** of the plurality of ETMs **118** as a voltage-supplied ETM and not to the other ETM **118A, 118B** as a voltage-deprived ETM. Thus, the supplied ETM heats or cools a respective first or second surface component **150, 152** based on the polarity of the voltage supplied as described herein while the voltage-deprive ETM neither provides heating nor cooling to a respective first or second surface component **150, 152.**

The first surface component **150** may be electrically connected to the second surface component **152** through a p-n couple **154.** The p-n couple **154** includes a p-type semiconductor in which charge carriers in the material are positive "holes" and an n-type semiconductor material in which charge carriers in the material are negative electrons. Current flow may be controlled in a direction based on an applied voltage polarity. By way of example, and not as a limitation, the p-n couple **154** includes an n-type semiconductor material joined to a p-type semiconductor material such that a positive voltage causes an ETM **118** to which the positive voltage is supplied to heat on one of the first surface component **150** and the second surface component **152** while a negative voltage supplied to the ETM **118** alternatively causes the ETM **118** to heat to the other of the first surface component **150** and the second surface component **152.**

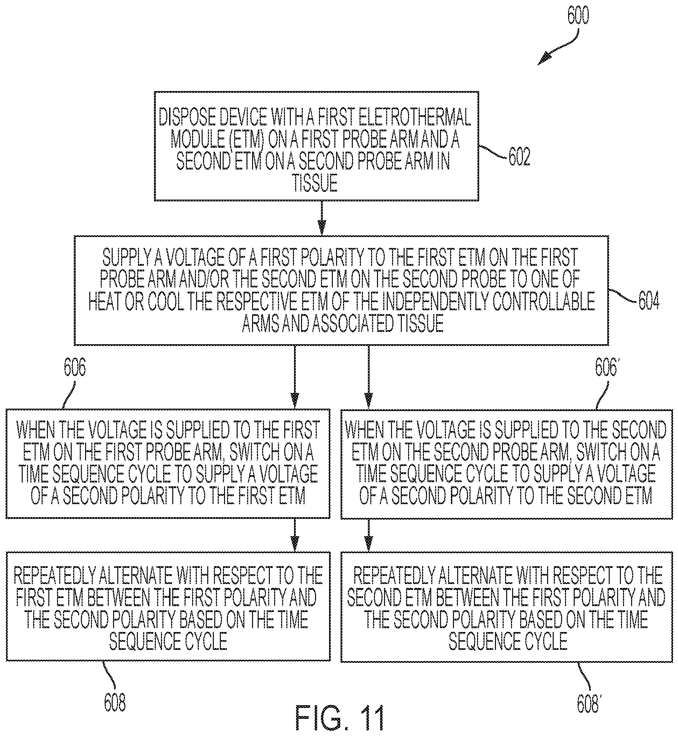

FIG. **11** illustrates a process **600** to use with the tumor ablation probe devices **100, 100', 200, 300, 400** described herein. The process **500** includes another method for tumor ablation with controlled precision of a temperature profile utilizing a tumor ablation probe device **100, 100', 200, 300, 400.** In block **602,** a distal end **106, 206, 306, 406** of the tumor ablation probe device **100, 100', 200, 300, 400** is disposed in a tissue **124.** By way of example and not as a limitation, the distal end **106, 206, 306, 406** includes at least one ETM **118A** on a first probe arm **116A** and at least one ETM **118B** on a second probe arm **116B,** each ETM **118A** and **118B** (FIG. **1****)** including a first surface component **150** and a second surface component **152** (FIG. **9****)** opposite and electrically connected to the first surface component **150.**

In block **604,** one of a first voltage of a first polarity and a second voltage of a second polarity opposite the first polarity is supplied via a circuit controller communicatively coupled to the tumor ablation probe device **100, 100', 200, 300, 400** to the at least one ETM **118A** on the first probe arm **116A,** the at least one ETM **118B** on the second probe arm **116B,** or both. In embodiments, the first polarity is configured to heat the first surface component **150** and cool the second surface component **152,** and the second polarity is configured to cool the first surface component **150** and heat the second surface component **152.** Each probe arm **116,** such as the first probe arm **116A** and the second probe arm **116B,** may be configured for independent control by the circuit controller.

In blocks **606, 606',** the other of the first voltage of the first polarity and the second voltage of the second polarity is supplied via the circuit controller to the at least one ETM **118A** on the first probe arm **116A,** the at least one ETM **118B** on the second probe arm **116B,** or both as one or more voltage-supplied ETMs, to repeatedly alter and switch the polarity based on a time sequence cycle. In blocks **608, 608'** the voltage is switched again with respect to the respective first or second ETM 118A, 118B with the other of the first voltage of the first polarity and the second voltage of the second polarity using the time sequence cycle. The, via the circuit controller, the process **600** involves a repeated alteration between the first polarity and the second polarity using the time sequence cycle.

In embodiments, each ETM **118** may be configured for independent control by the circuit controller, the first polarity is one of positive and negative and the second polarity is the other of positive and negative, and/or the first voltage may be equal to or different from the second voltage.

Referring to FIG. **12****,** a system **700** for implementing a computer and software-based method to utilize the tumor ablation probe devices of FIGS. **1-9** and the processes of FIGS. **10-11** is illustrated and may be implemented along with using a graphical user interface (GUI) that is accessible at a user workstation (e.g., a computer **724),** for example. The system **700** includes a communication path **702,** one or more processors **704,** a memory component **706,** a tumor ablation probe device **712** that may be any of the tumor ablation probe devices **100, 100', 200, 300, 400** described herein, a storage or database **714,** a switching component **716,** a network interface hardware **718,** a server **720,** a network **722,** and at least one computer **724.** The various components of the system **700** and the interaction thereof will be described in detail below.

In some embodiments, the system **700** is implemented using a wide area network (WAN) or network **722,** such as an intranet or the Internet, or other wired or wireless communication network that may include a cloud computing-based network configuration. The workstation computer **724** may include digital systems and other devices permitting connection to and navigation of the network. The lines depicted in FIG. **7** indicate communication rather than physical connections between the various components.

As noted above, the system **700** includes the communication path **702.** The communication path **702** may be formed from any medium that is capable of transmitting a signal such as, for example, conductive wires, conductive traces, optical waveguides, or other media capable of transmitting signals, or from a combination of media capable of transmitting signals. The communication path **702** communicatively couples the various components of the system **700.** As used herein, the term "communicatively coupled" means that coupled components are capable of exchanging data signals with one another such as, for example, electrical signals via conductive medium, electromagnetic signals via air, optical signals via optical waveguides, or other data signals via a corresponding data signal exchange medium.

As previously described, the system **700** includes the processor **704.** The processor **704** can be any device capable of executing machine readable instructions. Accordingly, the processor **704** may be a controller such as the circuit controller described herein, an integrated circuit, a microchip, a computer, or any other computing device. The processor **704** is communicatively coupled to the other components of the system **700** by the communication path **702.** Accordingly, the communication path **702** may communicatively couple any number of processors with one another, and allow the modules coupled to the communication path **302** to operate in a distributed computing environment. Specifically, each of the modules can operate as a node that may send and/or receive data. The processor **704** may process the input signals received from the system modules and/or extract information from such signals.

As previously described, the system **700** includes the memory component **706** coupled to the communication path **702** and communicatively coupled to the processor **704.** The memory component **706** may be a non-transitory computer readable medium or non-transitory computer readable memory and may be configured as a nonvolatile computer readable medium. The memory component **706** may comprise RAM, ROM, flash memories, hard drives, or any device capable of storing machine readable instructions such that the machine readable instructions can be accessed and executed by the processor **704.** The machine readable instructions may comprise logic or algorithm(s) written in any programming language such as, for example, machine language that may be directly executed by the processor, or assembly language, object-oriented programming (OOP), scripting languages, microcode, etc., that may be compiled or assembled into machine readable instructions and stored on the memory component **706.** Alternatively, the machine readable instructions may be written in a hardware description language (HDL), such as logic implemented via either a field-programmable gate array (FPGA) configuration or an application-specific integrated circuit (ASIC), or their equivalents. Accordingly, the methods described herein may be implemented in any conventional computer programming language, as pre-programmed hardware elements, or as a combination of hardware and software components. In embodiments, the system **700** may include the processor **704** communicatively coupled to the memory component **706** that stores instructions that, when executed by the processor **704,** cause the processor to perform one or more functions as described herein.

Still referring to FIG. **12****,** as previously described, the system **700** comprises the display such as a GUI on a screen of the computer **724** for providing visual output such as, for example, information, graphical reports, messages, or a combination thereof. The computer **724** may include one or more computing devices across platforms, or may be communicatively coupled to devices across platforms, such as mobile smart devices including smartphones, tablets, laptops, and/or other smart devices. The display can include any medium capable of transmitting an optical output such as, for example, a cathode ray tube, light emitting diodes, a liquid crystal display, a plasma display, or other optical output transmission mediums. Additionally, it is noted that the display or the computer **724** can include at least one of the processor **704** and the memory component **706.** While the system **700** is illustrated as a single, integrated system in FIG. **12****,** in other embodiments, the systems can be independent systems.

The system **700** comprises the tumor ablation probe device **712** as described herein to cause tumor necrosis via one or more ETMs **118** and the switching component **716** to cause the heating and cooling cycling to power the ETMs **118** to act to employ thermal energy to the tissue through a time sequence cycle causing a repeated alteration between voltage polarities to affect a thermal profile and thus to enhance tumor necrosis. The tumor ablation probe device **712** and the switching component **716** are coupled to the communication path **702** and communicatively coupled to the processor **704.** As will be described in further detail below, the processor **704** may process the input signals received from the system modules and/or extract information from such signals.

The system **700** includes the network interface hardware **718** for communicatively coupling the system **700** with a computer network such as network **722.** The network interface hardware **718** is coupled to the communication path **702** such that the communication path **702** communicatively couples the network interface hardware **718** to other modules of the system **700.** The network interface hardware **718** can be any device capable of transmitting and/or receiving data via a wireless network. Accordingly, the network interface hardware **718** can include a communication transceiver for sending and/or receiving data according to any wireless communication standard. For example, the network interface hardware **718** can include a chipset (e.g., antenna, processors, machine readable instructions, etc.) to communicate over wired and/or wireless computer networks such as, for example, wireless fidelity (Wi-Fi), WIMAX, BLUETOOTH, IRDA, WIRELESS USB, Z-WAVE, ZIGBEE, or other chipsets.

Still referring to FIG. **12****,** data from various applications running on computer **724** can be provided from the computer **724** to the system **700** via the network interface hardware **318.** The computer **724** can be any device having hardware (e.g., chipsets, processors, memory, etc.) for communicatively coupling with the network interface hardware **718** and a network **722.** Specifically, the computer **724** can include an input device having an antenna for communicating over one or more of the wireless computer networks described above.

The network **722** can include any wired and/or wireless network such as, for example, wide area networks, metropolitan area networks, the Internet, an intranet, the cloud, satellite networks, or other networks. Accordingly, the network **722** can be utilized as a wireless access point by the computer **724** to access one or more servers (e.g., a server **720).** The server **720** and any additional servers generally include processors, memory, and chipset for delivering resources via the network **722.** Resources can include providing, for example, processing, storage, software, and information from the server **720** to the system **700** via the network **722.** Additionally, it is noted that the server **720** and any additional servers can share resources with one another over the network **722** such as, for example, via the wired portion of the network, the wireless portion of the network, or combinations thereof.

It is noted that the terms "substantially" and "about" and "approximately" may be utilized herein to represent the inherent degree of uncertainty that may be attributed to any quantitative comparison, value, measurement, or other representation. These terms are also utilized herein to represent the degree by which a quantitative representation may vary from a stated reference without resulting in a change in the basic function of the subject matter at issue.

While particular embodiments have been illustrated and described herein, it should be understood that various other changes and modifications, which fall under the scope of the appended claims, may be made.

## Claims

1. A system for tumor ablation with controlled precision of a temperature profile utilizing a tumor ablation probe device (100, 200, 300, 400), the system comprising:
the tumor ablation probe device (100, 200, 300, 400) including a distal end (106, 206, 306, 406), the distal end (106, 206, 306, 406) comprising a plurality of electrothermal modules, ETMs, (118) proximally disposed on a device surface of the tumor ablation probe device, each ETM (118) including a first surface component (150) and a second surface component (152) opposite and electrically connected to the first surface component (150); and
a circuit controller communicatively coupled to the tumor ablation probe device (100, 200, 300, 400) and a non-transitory computer storage medium,
wherein the non-transitory computer storage medium stores instructions that, when executed by the circuit controller, cause the system to:
supply, via the circuit controller, one of a first voltage of a first polarity and a second voltage of a second polarity opposite the first polarity to at least one ETM (118) of the plurality of ETMs (118), wherein when the first polarity is supplied, the at least one ETM (118) heats the first surface component (150) and cools the second surface component (152), and when the second polarity is supplied, the at least one ETM (118) cools the first surface component (150) and heats the second surface component (152); and
repeatedly alternate, via the circuit controller, between the first polarity and the second polarity based on a time sequence cycle, wherein each ETM (118) is configured for independent control by the circuit controller.

2. The system of claim 1, the non-transitory computer storage medium stores further instructions that, when executed by the circuit controller, cause the system to supply one of the first voltage of the first polarity and the second voltage of the second polarity to the at least one ETM (118) of the plurality of ETMs (118) and not to at least one other ETM (118) of the plurality of ETMs (118).

3. The system of claim 1, wherein the distal end (106, 206, 306, 406) includes the at least one ETM (118) of the plurality of ETMs (118) on a first probe arm (116) and at least other one ETM (118) of the plurality of ETMs (118) on a second probe arm (116), and each of the first probe arm (116) and the second probe arm (116) is configured for independent control by the circuit controller.

4. The system of claim 1, wherein the first polarity is positive and the second polarity is negative.

5. The system of claim 1, wherein the first polarity is negative and the second polarity is positive.

6. The system of claim 1, wherein the first voltage is equal to the second voltage.

7. The system of claim 1, wherein the first voltage is different from the second voltage.

8. The system of claim 1, wherein the time sequence cycle is from about 2 seconds to about 5 seconds.

9. The system of claim 1, wherein when the first polarity is supplied, the at least one ETM (118) heats the first surface component (150) to a range from about 45 degrees Celsius to about 50 degrees Celsius, and when the second polarity is supplied, the at least one ETM (118) cools the first surface component (150) to about -10 degrees Celsius.

10. The system of claim 1, wherein the first surface component (150) of each ETM (118) is electrically connected to the second surface component (152) of each respective ETM (118) through a p-n couple.

## Patentansprüche

1. System zur Tumorablation mit kontrollierter Präzision eines Temperaturprofils unter Verwendung einer Tumorablationssondenvorrichtung (100, 200, 300, 400), umfassend:
die Tumorablationssondenvorrichtung (100, 200, 300, 400), einschließlich eines distalen Endes (106, 206, 306, 406), wobei das distale Ende (106, 206, 306, 406) eine Vielzahl von elektrothermischen Modulen, ETMs, (118) umfasst, die proximal auf einer Vorrichtungsoberfläche der Tumorablationssondenvorrichtung angeordnet sind, wobei jedes ETM (118) eine erste Oberflächenkomponente (150) und eine zweite Oberflächenkomponente (152) einschließt, die der ersten Oberflächenkomponente (150) gegenüberliegt und elektrisch mit ihr verbunden ist; und
eine Schaltungssteuerung, die kommunikativ mit der Tumorablationssondenvorrichtung (100, 200, 300, 400) und einem nichtflüchtigen Computerspeichermedium gekoppelt ist,
wobei das nichtflüchtige Computerspeichermedium Anweisungen speichert, die, wenn sie von der Schaltungssteuerung ausgeführt werden, das System zu Folgendem veranlassen:
Zuführen, über die Schaltungssteuerung, einer ersten Spannung einer ersten Polarität und einer zweiten Spannung einer zweiten Polarität, die der ersten Polarität entgegengesetzt ist, an mindestens ein ETM (118) aus der Vielzahl von ETMs (118), wobei, wenn die erste Polarität angelegt wird, das mindestens eine ETM (118) die erste Oberflächenkomponente (150) erwärmt und die zweite Oberflächenkomponente (152) kühlt, und wenn die zweite Polarität angelegt wird, das mindestens eine ETM (118) die erste Oberflächenkomponente (150) kühlt und die zweite Oberflächenkomponente (152) erwärmt; und
wiederholt Abwechseln, über die Schaltungssteuerung, zwischen der ersten Polarität und der zweiten Polarität basierend auf einem Zeitsequenzzyklus, wobei jedes ETM (118) für eine unabhängige Steuerung durch die Schaltungssteuerung ausgelegt ist.

2. System nach Anspruch 1, wobei das nichtflüchtige Computerspeichermedium weitere Anweisungen speichert, die, wenn sie von der Schaltungssteuerung ausgeführt werden, bewirken, dass das System eine der ersten Spannung der ersten Polarität und der zweiten Spannung der zweiten Polarität an mindestens ein ETM (118) der Vielzahl von ETMs (118) und nicht an mindestens ein anderes ETM (118) der Vielzahl von ETMs (118) zuführt.

3. System nach Anspruch 1, wobei das distale Ende (106, 206, 306, 406) mindestens ein ETM (118) der Vielzahl von ETMs (118) auf einem ersten Sondenarm (116) und mindestens ein weiteres ETM (118) der Vielzahl von ETMs (118) auf einem zweiten Sondenarm (116) einschließt, und sowohl der erste Sondenarm (116) als auch der zweite Sondenarm (116) für eine unabhängige Steuerung durch die Schaltungssteuerung ausgelegt sind.

4. System nach Anspruch 1, wobei die erste Polarität positiv und die zweite Polarität negativ ist.

5. System nach Anspruch 1, wobei die erste Polarität negativ und die zweite Polarität positiv ist.

6. System nach Anspruch 1, wobei die erste Spannung gleich der zweiten Spannung ist.

7. System nach Anspruch 1, wobei die erste Spannung von der zweiten Spannung verschieden ist.

8. System nach Anspruch 1, wobei der Zeitsequenzzyklus etwa 2 Sekunden bis etwa 5 Sekunden beträgt.

9. System nach Anspruch 1, wobei, wenn die erste Polarität geliefert wird, das mindestens eine ETM (118) die erste Oberflächenkomponente (150) auf einen Bereich von etwa 45 Grad Celsius bis etwa 50 Grad Celsius erwärmt und, wenn die zweite Polarität geliefert wird, das mindestens eine ETM (118) die erste Oberflächenkomponente (150) auf etwa -10 Grad Celsius abkühlt.

10. System nach Anspruch 1, wobei die erste Oberflächenkomponente (150) jedes ETM (118) mit der zweiten Oberflächenkomponente (152) jedes entsprechenden ETM (118) über eine p-n-Kopplung elektrisch verbunden ist.

## Revendications

1. Système pour ablation de tumeur à précision commandée d'un profil de température utilisant un dispositif (100, 200, 300, 400) de sonde d'ablation de tumeur, le système comprenant :
le dispositif (100, 200, 300, 400) de sonde d'ablation de tumeur incluant une extrémité distale (106, 206, 306, 406), l'extrémité distale (106, 206, 306, 406) comprenant une pluralité de modules électrothermiques, ETMs, (118) disposés de manière proximale sur une surface de dispositif du dispositif de sonde d'ablation de tumeur, chaque ETM (118) incluant un premier composant de surface (150) et un deuxième composant de surface (152) opposé et électriquement connecté au premier composant de surface (150) ; et
un dispositif de commande de circuit couplé en communication au dispositif (100, 200, 300, 400) de sonde d'ablation de tumeur et à un support de stockage informatique non transitoire,
dans lequel le support de stockage informatique non transitoire stocke des instructions qui, lorsqu'elles sont mises en oeuvre par le dispositif de commande de circuit, amènent le système à :
apporter, via le dispositif de commande de circuit, l'une parmi une première tension d'une première polarité et une deuxième tension d'une deuxième polarité opposée à la première polarité à au moins un ETM (118) de la pluralité d'ETM (118), dans lequel, lorsque la première polarité est apportée, le au moins un ETM (118) chauffe le premier composant de surface (150) et refroidit le deuxième composant de surface (152), et lorsque la deuxième polarité est apportée, le au moins un ETM (118) refroidit le premier composant de surface (150) et chauffe le deuxième composant de surface (152) ; et
alterner de façon répétée, via le dispositif de commande de circuit, entre la première polarité et la deuxième polarité sur la base d'un cycle séquentiel temporel, dans lequel chaque ETM (118) est configuré pour une commande indépendante par le dispositif de commande de circuit.

2. Système selon la revendication 1, le support de stockage informatique non transitoire stocke d'autres instructions qui, lorsqu'elles sont mises en oeuvre par le dispositif de commande de circuit, amènent le système à apporter l'une parmi la première tension de la première polarité et la deuxième tension de la deuxième polarité au au moins un ETM (118) de la pluralité d'ETM (118) et pas à au moins un autre ETM (118) de la pluralité d'ETM (118).

3. Système selon la revendication 1, dans lequel l'extrémité distale (106, 206, 306, 406) inclut le au moins un ETM (118) de la pluralité d'ETM (118) sur un premier bras (116) de sonde et au moins un autre ETM (118) de la pluralité d'ETM (118) sur un deuxième bras (116) de sonde, et chacun du premier bras (116) de sonde et du deuxième bras (116) de sonde est configuré pour une commande indépendante par le dispositif de commande de circuit.

4. Système selon la revendication 1, dans lequel la première polarité est positive et la deuxième polarité est négative.

5. Système selon la revendication 1, dans lequel la première polarité est négative et la deuxième polarité est positive.

6. Système selon la revendication 1, dans lequel la première tension est égale à la deuxième tension.

7. Système selon la revendication 1, dans lequel la première tension est différente de la deuxième tension.

8. Système selon la revendication 1, dans lequel le cycle séquentiel temporel fait d'environ 2 secondes à environ 5 secondes.

9. Système selon la revendication 1, dans lequel, lorsque la première polarité est apportée, le au moins un ETM (118) chauffe le premier composant de surface (150) à une plage allant d'environ 45 degrés Celsius à environ 50 degrés Celsius, et lorsque la deuxième polarité est apportée, le au moins un ETM (118) refroidit le premier composant de surface (150) à environ -10 degrés Celsius.

10. Système selon la revendication 1, dans lequel le premier composant de surface (150) de chaque ETM (118) est électriquement connecté au deuxième composant de surface (152) de chaque ETM (118) respectif par le biais d'un couple p-n.
